# EUROPEAN PATENT APPLICATION

(11) **EP 2 371 808 A1**
(43) Date of publication of application: **05.10.2011**
(21) Application number: 10155797.3
(22) Date of filing: 08.03.2010
(51) Int. Cl.: C07C 69/92, C07D 307/80

(54) **Process for preparing dronedarone**

(71) Applicant: Ratiopharm GmbH, 89079 Ulm (DE)
(72) Inventor: STOHANDL, Jiri, 59255 Bobrova (CZ); SINDELAROVA, Jana, 798 45 Jednov (CZ)
(74) Representative: Teipel, Stephan

(57) **Abstract**

The present invention relates to a process for preparing dronedarone and in particular for preparing an intermediate useful in the preparation of dronedarone. Furthermore, the present invention relates to a process for preparing crystalline dronedarone hydrochloride as well as pharmaceutical compositions comprising this salt.

## Description

The present invention relates to a process for preparing dronedarone and in particular for preparing an intermediate useful in the preparation of dronedarone. Furthermore, the present invention relates to a process for preparing crystalline dronedarone hydrochloride as well as pharmaceutical compositions comprising this salt.

Dronedarone (N-[2-butyl-3-[4-[3-(dibutylamino)propoxy]benzoyl]-5-benzofuranyl]methane-sulfonamide) has the following chemical structure:

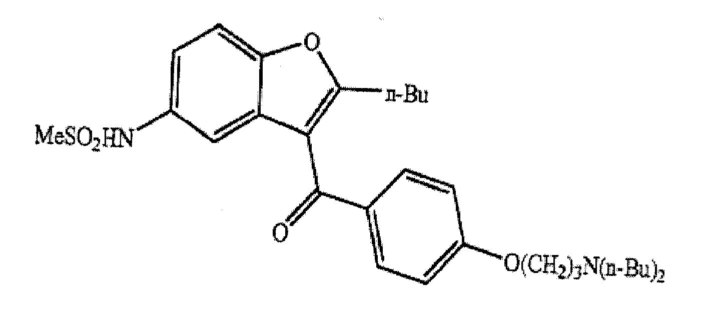

From EP 0 471 609 A2 dronedarone is known as a drug for the treatment of arrhythmia. This document also discloses a method for preparing dronedarone as well as a method for preparing dronedarone hydrochloride. According to this method, first dronedarone free base is prepared and the crude product thus obtained is purified by elution chromatography on a silica column. The purified dronedarone is then dissolved in anhydrous ethyl acetate. A solution of hydrochloric acid in ether is added and the hydrochloride salt is precipitated.

In improved process for the preparation of dronedarone is disclosed in US 7,312,345. One of the intermediates required in this process is the methyl 4-(3-bromopropoxy)benzoate [17a] which is obtained by reacting methyl 4-hydroxybenzoate [16a] with 1,3-dibromopropane. This reaction results in a mixture of the desired methyl 4-(3-bromopropoxy)benzoate [17a] and the undesired by-product dimethyl 4,4'-[1,3-propanediylbis(oxy)]bisbenzoate [20]. The methyl 4-(3-bromopropoxy)benzoate [17a] is then converted into the next intermediate methyl 4-(3-dibutylaminopropoxy)benzoate [18a]. This prior art process is shown in the following reaction scheme:

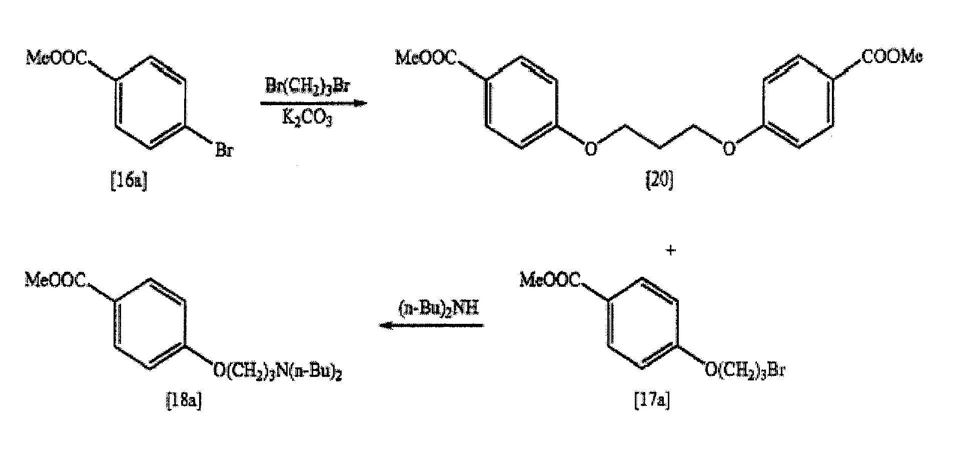

According to US 7,312,345, the mixture of the desired product [17a] und the undesired by-product [20] obtained in the first reaction step is used for the second reaction step thereby obtaining compound [18a]. The yield in the second reaction step is, however, only 61.1 %.

Consequently, there is a need for a process for the preparation of methyl 4-(3-dibutylaminopropoxy)benzoate which does not result in any undesired by-products and which results in a higher overall yield in the following reaction step.

It has now surprisingly been found that if the reaction of methyl 4-hydroxybenzoate is carried out with 1-bromo-3-chloropropane instead of 1,3-dibromopropane, methyl 4-(3-chloropropoxy)benzoate is obtained in high yield and in a purity which allows immediate use of the product in the following reaction steps. Moreover, the yield of the conversion of the thus obtained methyl 4-(3-chloropropoxy)benzoate into the methyl 4-(3-dibutylaminopropoxy)benzoate is about 90 %, thus being considerably higher than the yield of 61.1 % in the prior art process.

Thus, the present invention relates to a process for preparing dronedarone or a pharmaceutically acceptable salt thereof, which process comprises the step of reacting a protected 4-hydroxybenzoic acid of the formula II 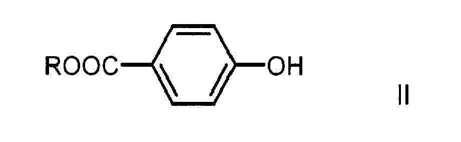 wherein R is a carboxyl protecting group, with 1-bromo-3-chloropropane, to obtain a protected 4-(3-chloropropoxy)benzoic acid of the formula I 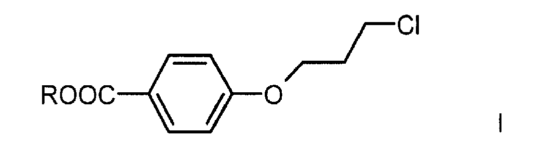 wherein R is defined as above.

As protecting group R any group conventionally used as protecting group for carboxyl groups can be used. Preferred protecting groups are linear, branched or cyclic C₁₋₁₀ alkyl groups, such as methyl, ethyl, n-propyl, iso-propyl, n-butyl and tert-butyl. Most preferred is methyl.

In one embodiment of the present invention the above reaction is carried out in the presence of potassium carbonate in methyl ethyl ketone (MEK) as solvent. For example, the reaction can be carried out while stirring under reflux conditions for several hours, such as about six hours. The cold mixture can then be filtered off and the solids can, for example, be washed with MEK. The combined filtrates can be evaporated under reduced pressure and increased temperature, such as at about 90°C. The yield of this reaction is about 96 % and the product does not require any purification before its further reactions to dronedarone.

In a preferred embodiment the process for preparing dronedarone comprises the further steps of
a) reacting the compound of the formula I 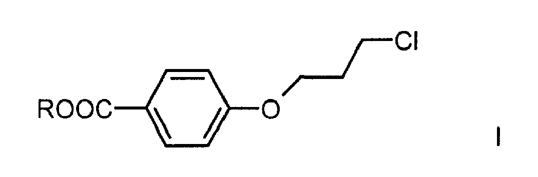 wherein R is a carboxyl protecting group, with dibutylamine to obtain a protected 4-(3-dibutylaminopropoxy)benzoic acid of the formula III 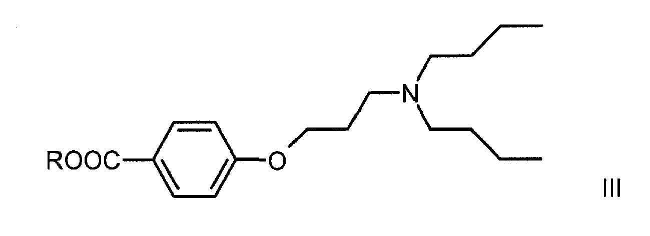 wherein R is defined as above;
b) deprotecting the compound of the formula III;
c) converting the deprotected compound obtain in step b) into 4-(3-dibutylaminopropoxy)benzoyl chloride of the formula IV 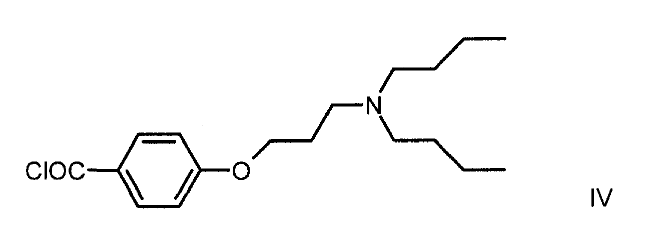 or a pharmaceutically acceptable salt thereof; and
d) reacting the compound of the formula IV or a pharmaceutically acceptable salt thereof with N-(2-butylbenzofuran-5-yl)methanesulfonamide of the formula V 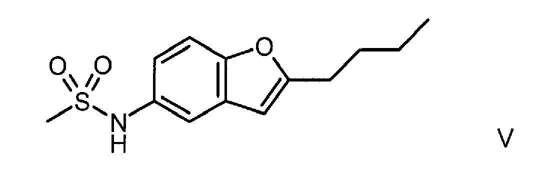
to obtain dronedarone or a pharmaceutically acceptable salt thereof.

Above step a) can, for example, be carried out in the presence of potassium iodide in DMF as the solvent. Surprisingly, the reaction of the present invention in step a) yields about 90 % of the compound of formula III while the prior art process known from US 7,312,345 has a much lower yield of only 61.1 %.

The deprotection of the compound of the formula III in above step b) can be carried out either in aqueous hydrochloric acid solution or in an aqueous base, such as sodium hydroxide. Preferably, the reaction takes place in the presence of sodium hydroxyide.

Above step c) can be carried out using thionyl chloride in a suitable solvent, such as chlorobenzene.

Above step d) can be carried out by Friedel-Crafts acylation reaction in the presence a Lewis acid. Suitable Lewis acids are, for example, FeCl₃, SnCl₄ and AlCl₃. Aluminium chloride being preferred. The reaction can, for example, be carried out in methylene chloride at a temperature slightly above 0°C. Preferably, the dronedarone is isolated from the reaction mixture as dronedarone hydrochloride. It forms a brown amorphous solidifying foam after evaporation of the solvent.

N-(2-Butylbenzofuran-5-yl)methansulfonamide of the formula V required in above step d) can be prepared by the process disclosed in US 7,312,345. The overall reaction is shown in the following reaction scheme:

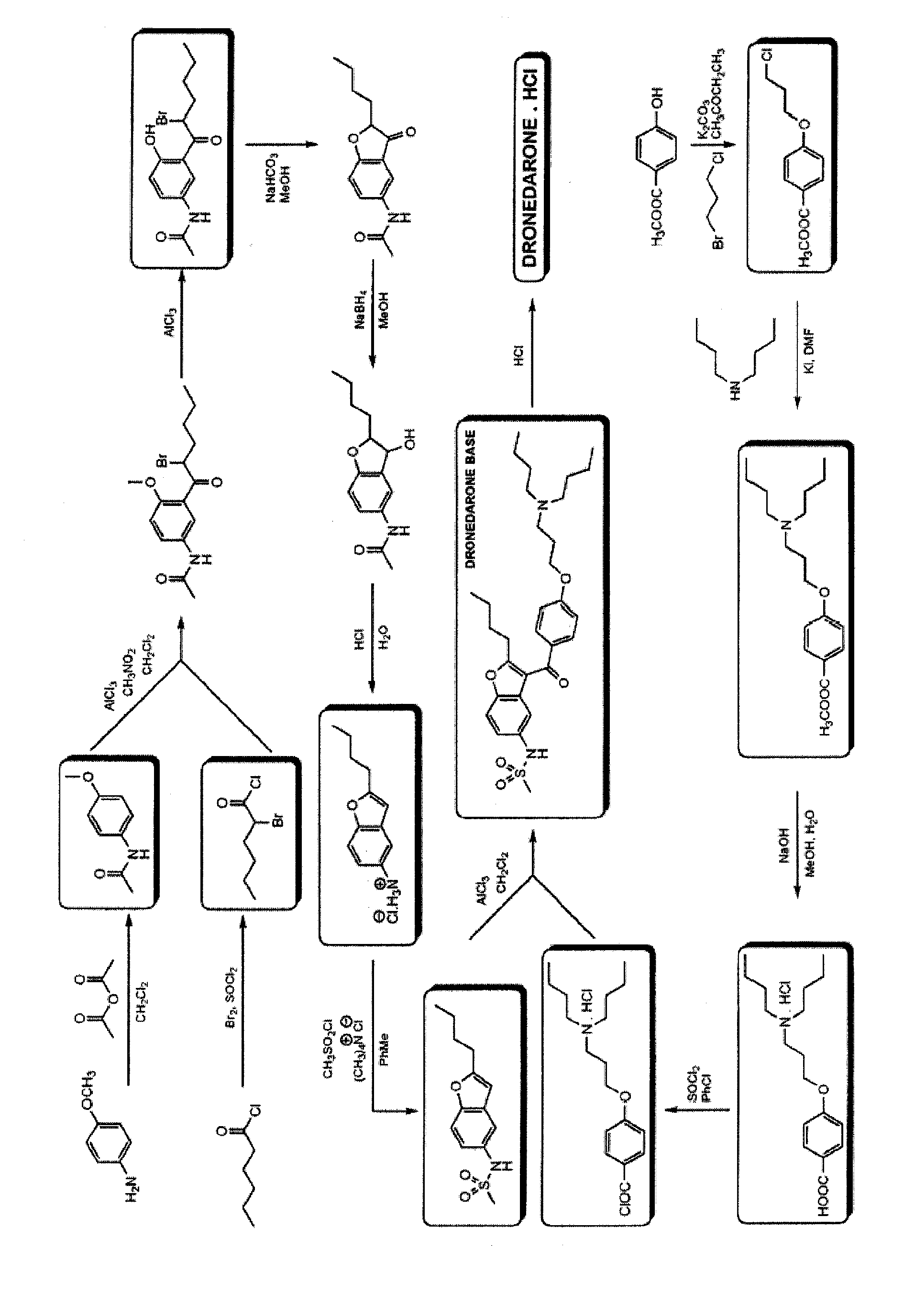

Extensive studies were carried out for converting the amorphous dronedarone hydrochloride into crystalline dronedarone hydrochloride. Besides the above-described method of preparing dronedarone hydrochloride as disclosed in EP 0 471 609 A2 a further method for the preparation of dronedarone hydrochloride is disclosed in US 2004/0048921 A1. According to this method, dronedarone hydrochloride is first obtained in crude from. From this product dronedarone free base is obtained in crude form and finally the dronedarone free base in crude form is converted into dronedarone hydrochloride using isopropanol as solvent.

When repeating the prior methods for preparing dronedarone hydrochloride, it was surprisingly found that none of these methods led to any crystalline product. Only brown sticky amorphous clot could be isolated. Variation of concentration and temperature did not bring the desired result. Also the use of further different solvents such as diethyl ether and acetone instead of ethyl acetate and isopropanol was unsuccessful. Furthermore, even recrystallization of the sticky gum from other solvents, such as isopropanol, ethyl acetate, ethyl acetate containing water, isopropyl acetate and acetone, was unsuccessful. Only amorphous powders could be obtained.

Further investigations revealed that the dronedarone free base purified by elution chromatography on a silica column according to the prior art methods still contains small amounts of impurities, which prevent the dronedarone salts from crystallizing. In was in particular found that dronedarone free base resulting from the synthesis contained oligomeric impurities not detected by HPLC that made impossible successful crystallization of any salt.

It was now found that this problem can be overcome by further purification of the dronedarone free base before preparing the desired salt. If the dronedarone free base has a very high chemical purity and in particular is substantially free of impurities having a molecular weight being higher than the molecular weight of dronedarone free base, such as oligomeric impurities, the hydrochloride salt of dronedarone readily crystallizes from its solution. Thus, the present invention also provides a crystalline dronedarone hydrochloride.

The hydrochloride salt of the present invention is considered as crystalline if at least 80 % by weight of the total salt is in crystalline form, preferably at least 90 %, more preferably at least 95 %, such as at least 99 %. Most preferably the salt of the present invention is substantially in crystalline form and in particular more than 99.5 % by weight, such as 100 % by weight of the total salt is crystalline. The crystallinity of the salt can be determined, for example, by XRPD.

Preferably, the hydrochloride salt of the present invention has a chemical purity of > 99 %, more preferably of > 99.5 %, such as ≥ 99.7 % and in particular ≥ 99.8 %. The chemical purity of the salt is determined by HPLC.

In a particularly preferred embodiment the hydrochloride salt of the present invention is substantially free of impurities having a molecular weight being higher than the molecular weight of dronedarone free base, such as oligomers or polymers being obtained as undesired by-products in the preparation of the dronedarone. "Substantially free" means that the salt contains no or as little as possible of the high molecular weight impurities. Preferably, the amount of the high molecular weight impurities is such as obtained when adding diethyl ether to crude dronedarone free base in a weight ratio of dronedarone free base to diethyl ether of about 1:40, stirring for 30 minutes at 23°C, filtering off the precipitated impurities and recovering the dronedarone free base from the solution.

The present invention furthermore relates to a new polymorphic form of dronedarone hydrochloride. The polymorphic form of the crystalline salt is defined by characteristic peaks of its XRPD pattern. The XRPD pattern was measured under the following conditions:
- Instrument:: XRD-7 Seifert Company
- Wavelength:: CuK alfa1 radiation
- Detector:: Linear PSD
- Monochromator:: Primary Ge (111) in the reflection mode
- Scan Mode:: Transmittance / Moving PSD / Moving omega
- Scan Type:: 2Theta: Omega

Alternatively, the polymorphic form of the crystalline salt is characterized by its melting point as measured by DSC using a Perkin-Elmer DSC7 apparatus at a scanning rate of 10.0°C/min. As pelting point the peak in the DSC is defined.

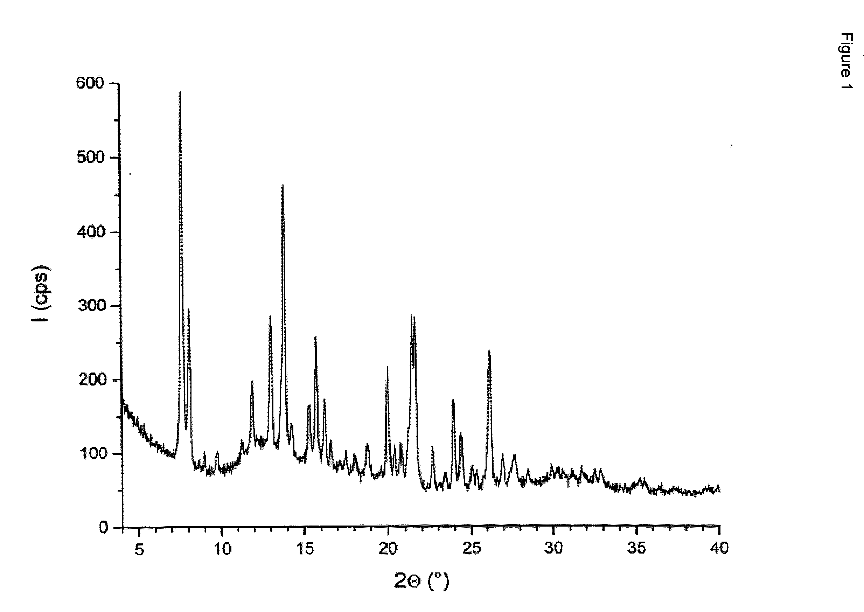

Crystalline dronedarone hydrochloride in the polymorphic form of the present invention shows an XRPD pattern having characteristic peaks at 7.7 ± 0.2, 13.8 ± 0.2, 21.7 ± 0.2 and 26.2 ± 0.2 degree 2-Theta. Preferably, the XRPD pattern has characteristic peaks at 7.7 ± 0.2, 13.0 ± 0.02, 13.8 ± 0.02, 15.7 ± 0.02, 21.4 ± 0.02, 21.7 ± 0.02 and 26.2 ± 0.02 degree 2-Theta. The XRPD pattern of crystalline dronedarone hydrochloride of the present invention is shown in figure 1. The most intense peaks of this XRPD pattern are summarized in the following table 1.

**Table 1**

| **Degree 2-Theta (±0.2)** | **Relative Intensity** |
|---|---|
| 7.7 | 100 |
| 8.1 | 45 |
| 9.0 | 6 |
| 9.7 | 6 |
| 11.3 | 6 |
| 11.9 | 26 |
| 13.0 | 45 |
| 13.8 | 84 |
| 14.3 | 13 |
| 15.3 | 20 |
| 15.7 | 40 |
| 16.2 | 22 |
| 16.6 | 11 |
| 17.5 | 8 |
| 18.1 | 7 |
| 18.8 | 11 |
| 20.0 | 34 |
| 20.5 | 12 |
| 20.8 | 13 |
| 21.4 | 47 |
| 21.7 | 50 |
| 22.7 | 11 |
| 23.4 | 5 |
| 24.0 | 27 |
| 24.4 | 17 |
| 25.0 | 8 |
| 25.3 | 7 |
| 26.2 | 41 |
| 26.9 | 12 |
| 27.7 | 11 |
| 28.5 | 8 |
| 29.9 | 9 |
| 30.6 | 8 |
| 31.1 | 8 |
| 31.7 | 8 |
| 32.5 | 8 |
| 32.9 | 8 |

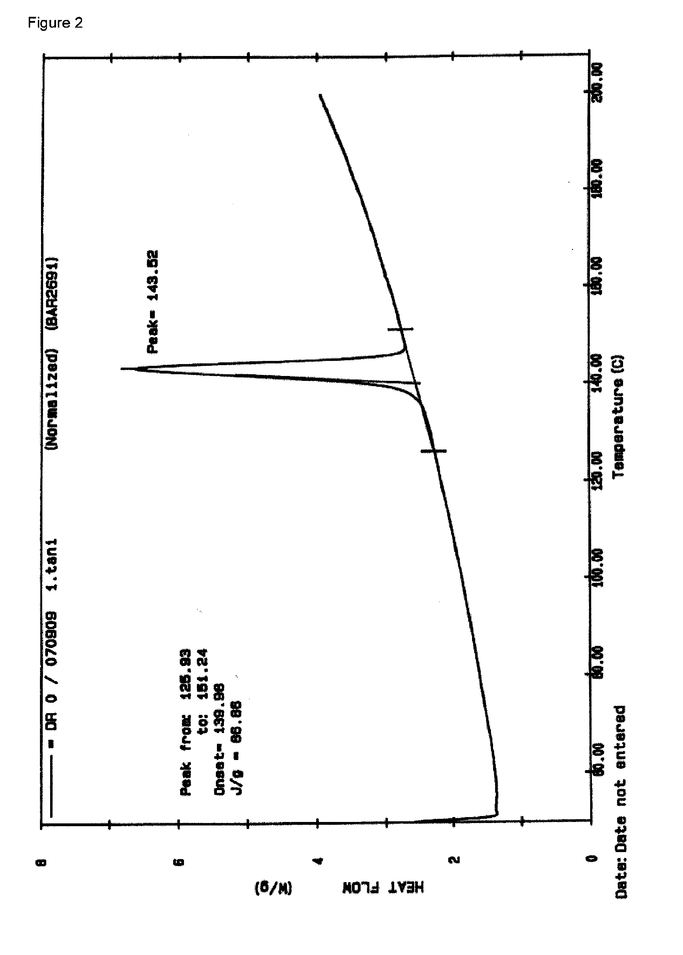

Crystalline dronedarone hydrochloride of the present invention has a melting temperature of 144 ± 1°C. The DSC of this compound is shown in figure 2.

As discussed above, it was surprisingly found that the batch obtained in the synthesis of dronedarone free base contains impurities having a molecular weight being higher than the molecular weight of dronedarone free base, such as oligomers and polymers obtained as by-products in the reaction sequence. Since these impurities prevent the final product from crystallization, it is desirable to remove these impurities from the reaction product. Therefore, the present invention also relates to the above-described process which comprises the additional step of essentially removing those impurities having a molecular weight being higher than the molecular weight of dronedarone free base.

In one embodiment of this process said impurities are removed by adding a solvent to the batch of dronedarone free base, dissolving the dronedarone free base in the solvent and removing the precipitated impurities, for example, by filtration. As solvent any solvent dissolving the dronedarone free base but not dissolving the high molecular weight impurities can be use. Suitable solvents are ethers and in particular diethyl ether. The amount of solvent is not particulary limited but it has been found that good results are obtained if the solvent is used in a high excess relative to the dronedarone free base. Suitably, the solvent is used in an amount such that the weight of the dronedarone free base relative to the weight of the solvent is in the range of 1:20 to 1:60, preferably in the range of 1:30 to 1:50, such as about 1:40.

In one embodiment of the process of the present invention about one part by weight of dronedarone free base is added to about 40 parts by weight of diethyl ether and stirred for about 30 minutes at 23°C. The precipitated high molecular weight impurities are filtered off using, for example, a filter paper, and the filtrate containing the purified dronedarone free base is evaporated.

To additionally remove the remaining impurities from the dronedarone free base obtained in the above purification process, a further purification step using, for example, silica gel can be employed. For this second purification step the dronedarone free base can be dissolved in a suitable solvent, such as dichloromethane. To this solution silica gel can be added and the suspension can be stirred at room temperature for a time sufficient to remove at least part of the remaining impurities. The silica gel can then be filtered off and the filtrate can be evaporated to obtain the purified dronedarone free base.

To prepare the salt of the present invention from the dronedarone free base the purified dronedarone free base can subsequently be converted into the crystalline dronedarone hydrochloride by mixing a solution of dronedarone free base with hydrochloric acid, precipitating the crystalline salt and collecting the precipitate, for example, by filtration. Thus, the present invention also relates to a process for the preparation of crystalline dronedarone hydrochloride, which process comprises any of the above-described purification processes and the subsequent preparation of the crystalline dronedarone hydrochloride from the dronedarone free base obtained in the purification process.

Due to its crystallinity the salt of the present invention is particularly suited for the preparation of pharmaceutical compositions. Therefore, the present invention also relates to a pharmaceutical composition comprising crystalline hydrochloride. Suitable pharmaceutical compositions are known to a person skilled in the art and described in the above-referenced prior art documents.

Furthermore, the present invention relates to a protected 4-(3-chloropropoxy)benzoic acid of the above formula I wherein R is a carboxyl protecting group as defined above. This compound is an intermediate in the subject process for preparing dronedarone. Therefore, the present invention also relates to the use of the protected 4-(3-chloropropoxy)benzoic acid of the formula I in the preparation of dronedarone or a pharmaceutically acceptable salt thereof.

The attached figures show in
figure 1 the XRPD pattern of crystalline dronedarone hydrochloride, and
figure 2 the DSC of crystalline dronedarone hydrochloride.

The invention will now be explained by the following examples which are not intended to be construed as limiting.

### Examples

Dronedarone free base as used in the following examples was prepared as described above.

### Example 1 - Precipitation of impurities in ether

Base resulting from the synthesis (HPLC purity approx. 90 %) contained oligomeric impurities not detected by HPLC that made impossible successful crystallization of any salt.

Dronedarone base (320 g) was dissolved in ether (12 L) and stirred for 30 minutes at laboratory temperature. Precipitated amorphous oligomeric impurities were filtered off through filter paper and yellowish filtrate was evaporated on Rotary Vacuum Evaporator (RVE).

Yield was 271.7 g (88 %) HPLC purity was 90%.

### Example 2 - Purification on silica gel

Dronedarone base (273.9 g) was dissolved in tenfold volume (2739 mL) of dichloromethane and the same amount of silica gel (273.9 g) was added. Suspension was stirred for one hour at laboratory temperature and then silica gel was filtered off and washed with dichloromethane (2739 mL) and mixture of dichloromethane and methanol 1:1 (2739 mL). Combined filtrates were evaporated on RVE.

Yield was 238.7 g (87 %), HPLC purity was 92 %.

### Example 3 - Precipitation of mesylate

Dronedarone base purified according to Example 1 and Example 2 (117.0 g; 0.21 mol) was dissolved in acetone and the solution was then cooled down to ―20°C. Solution of methanesulphonic aicd (20.2 g; 13.6 mL; 0.21 mol) in acetone (211 mL) was added dropwise. Precipitated white suspension was stirred for one hour while the temperature was maintained bewenn ―20 and ―10°C. Precipitate was filtered off through sintered glass under nitrogen and washed twice with ether cooled to 0°C (500 mL). Product was dried at 40°C and 133 Pa.

Yield was 99.6 g (85 %), HPLC purity was 98 %.

### Example 4 - Preparation of dronedarone base from mesylate

Dronedarone mesylate (150 g; 0.23 mol) was dissolved in dichloromethane and the solution was neutralized with saturated solution of NaHCO₃ (approx. 100 mL). Organic layer was separated and water phase was washed with dichloromethane (150 mL). Combined organic layers were dried over anhydrous sodium sulfate and then evaporated on RVE.

Yield was 128 g (almost theoretical), HPLC purity was 97.4 %.

### Example 5 - Dronedarone hydrochloride

Dronedarone base (128.9 g; 0.23 mol) was dissolved in 2-butanone (1280 mL) and the solution was cooled down to ―20°C. Hydrochloric acid (0.25 mol; 50 mL of 5M HCl in ether) was added dropwise. Precipitated white suspension was stirred for one hour at ―20°C and then filtered off through sintered glass under nitrogen. Obtained crystal was washed with 2-butanone, cooled down to 0°C (200 mL) and then washed twice with ether cooled down to 0°C (500 mL).

Yield was 124.2 g (91 %), HPLC purity was 99.3 %.

### Example 6 - Recrystallization of dronedaron hydrochloride

Dronedarone hydrochloride (124.2 g; 0.21 mol) was dissolved in 2-butanone (800 mL) under reflux and then let to cool down to laboratory temperature and then cooled down to -10°C. Crystalline product was filtered off though sintered glass under nitrogen and washed with 2-butanone cooled down to 0°C (300 mL) and twice with ether cooled down to 0°C (500 mL). Product was dried at 40°C and 133 Pa.

Yield was 116.7 g (94 %), HPLC purity was 99.7 %.

Ratio of dronedarone base to hydrochloric acid was determined by argentometric titration. The determined ratio was 1:1.

### Example 7

Methyl-4-hydroxybenzoate (300 g; 1.97 mol); 1-bromo-3-chloro-propane (341.5 g; 213.4 mL, 1.1 eq.) and K₂CO₃ (300 g; 2.17 mol) was placed into 4 I glass reactor equipped with mechanical stirrer, thermometer, reflux and thermostatic bath. 2-butanone (1400 mL) was added. The mixture was warmed up and refluxed for nine hours. After three hours the conversion was 80 % (HPLC). After six hours the conversion was 93 % (HPLC). Then additional 1-bromo-3-chloro-propan (16.8 g; 0.05 eq.) was added. After nine hours the conversion was 97.5 % (HPLC). The reaction mixture was evaporated on RVE. The solid residue was extracted with water (2000 mL) and chloroform (2000 mL). Organic layer was separated and washed once with saturated brine and once with water. Then it was dried with Na₂SO₄ and evaporated.

Yield was 441.4 g (98 %) of crystalline substance.

### Example 8

The same experiment as in Example 7 was carried out in DMF and NMP. Conversion was 95.9 % (DMF) and 97 % (NMP) in two and half hour (DMF) and 1 hour (NMP). Reaction time was significantly shortened in comparison with 2-butanone.

Workup: The reaction mixture was diluted with four volumes of water, then CH₂Cl₂ (2000 mL) was added. The layers were separated, the water layer was extracted with CH₂Cl₂ (500 mL). Combined organic layers were washed three times with water, dried with Na₂SO₄ and evaporated to dryness.

Yield was 436.2 g (97 %) of crystalline substance in NMP and 432 g (95.9 %) in DMF.

### Example 9

Into 2000 mL three necked flask equipped with drying tube, mechanical stirrer, thermometer, reflux and thermostatic bath 4-(3-chloropropoxy)methylbenzoate (280.9 g; 1.23 mol), dibutylamine (229.4 ml; 1.35 mol) and K₂CO₃ (186.6; 1.35 mol) were charged into DMF (970 mL) and heated at 50 °C for twenty two hours. After four and half hours the conversion was 65 %, after twenty two hours the conversion was 97 %. Then the reaction mixture was diluted with water (4:1) and chloroform (2000 mL). After separation of phases the water layer was extracted once more with chloroform (250 mL). Combined organic layers were extracted twice with brine and once with water, then dried with Na₂SO₄. The solvent was evaporated.

Yield was 384.7 g (97 %) of yellow oil.
Confirmation: HR-MS, (M+H)⁺ = 322.2365

### Example 10

Into 2000 mL three necked flask equipped with drying tube, mechanical stirrer, thermometer, condenser and thermostatic bath 4-(3-chloropropoxy)methylbenzoate (280.9 g; 1.23 mol), dibutylamine (229.4 ml; 1.35 mol) and K₂CO₃ (186.6 g; 1.35 mol) were charged into reactor and NMP (970 mL) was added and then heated at 50 °C for 12 hours. After 2.5 hours the conversion was 58 %, after 14 hours the conversion was 97 %. Then the reaction mixture was diluted with water (3:1) and chloroform (2000 mL). After separation of phases the water layer was extracted with chloroform (250 mL). Combined organic layers were extracted twice with brine and once with water, then dried with Na₂SO₄. The solvent was evaporated.

Yield was 386.2 g (97.4 %) of yellow oil.
Confirmation: HR-MS, (M+H)⁺ = 322.2368

### Example 11

Protected 4-(3-dibutylaminopropoxy)benzoic acid (384.7 g; 1.2 mol) was dissolved in methanol (2677 mL). Solution of NaOH (239.3 g; 5.98 mol) in water (297 mL) was added and refluxed.

Workup: Reaction mixture was filtered and evaporated. The residue was dissolved in water (2500 mL) and product was precipitated with HCl 35 % (800 mL). After cooling down crystals were filtered and washed twice with water (2x 500 mL). Product was dried under vacuum (1-2 torr) at 50°C for eight hours.

Yield was 314.6 g (76 %), of white crystal
Confirmation: HR-MS, (M+H)⁺ = 308.2202

### Example 12

To the suspension of 4-(3-dibutylaminopropoxy)benzoic acid (3.1 g; 9.0 mmol) in chlorobenzene, DMF (1 drop) was added. SOCl₂ (2.1 g; 1.3 mL; 18.0 mmol; 2 eq.) in chlorobenzene was added dropwise at room temperature.

Workup: Chlorobenzene and SOCl₂ were distilled off (65°C, 100 torr). Maximum temperature of the bath is 85°C.

Yield: The acylchloride was used as such in Example 14 without isolation.

### Example 13

To the suspension of 4-(3-dibutylaminopropoxy)benzoic acid (68.1 g; 0.198 mol) in chlorobenzene and DMF, SOCl₂ (47.1 g; 28.7 mL; 0,396 mol; 2 eq.) in chlorobenzene was added dropwise at room temperature.

Isolation: Chlorobenzene and SOCl₂ were distilled off (65°C, 100 torr). Maximum temperature of the bath is 85 °C.

Yield: The acylchloride was used as such in Example 14 without isolation.

### Example 14

4-(3-Dibutylaminopropoxy)benzoyl chloride (71.8 g; 0.198 mol; 1.06 eq.) and N-(2-butylbenzofuran-5-yl)methanesulfonamide (50.0 g; 0.187 mol) were mixed in CH₂Cl₂ (350 mL) and cooled down to 0 °C. AlCl₃ (99.7 g; 0.748 mol; 4.0 eq) was added, during addition (two hours) the temperature was kept between 0 - 5 °C. Reaction mixture was kept between 0 - 10 °C for twenty hours.

Workup: Reaction mixture was poured on the mixture ice/HCl 1:1. Water was extracted once with 1000 mL and twice with 500 mL CH₂Cl_{2.} Organic layer was washed once with 1000 mL water and dried with Na₂SO₄. Solvent was evaporated on RVE. Product was transferred to the base: dissolved in CH₂Cl₂ and extracted with saturated solution of NaHCO₃ and dried with Na₂SO₄.

Yield was nearly quantitative as light brown foam.
Confirmation: HR-MS, (M+H)⁺ = 557.3043

## Claims

1. Process for preparing dronedarone or a pharmaceutically acceptable salt thereof, which process comprises the step of reacting a protected 4-hydroxybenzoic acid of the formula II 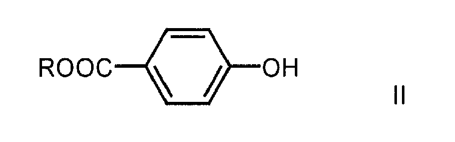 wherein R is a carboxyl protecting group, with 1-bromo-3-chloropropane, to obtain a protected 4-(3-chloropropoxy)benzoic acid of the formula I 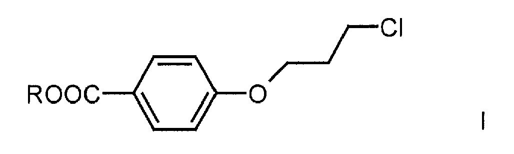 wherein R is defined as above.

2. Process according to claim 1, wherein the reaction is carried out in the presence of potassium carbonate in methyl ethyl ketone (MEK) as solvent.

3. Process according to claim 1 or 2, wherein R is methyl.

4. Process according to any of claims 1-3, comprising the further steps of
a) reacting the compound of the formula I 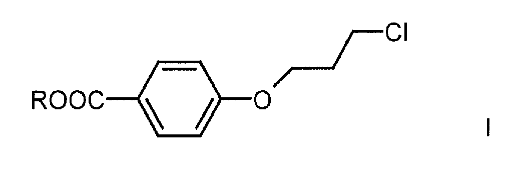 wherein R is a carboxyl protecting group, with dibutylamine to obtain a protected 4-(3-dibutylaminopropoxy)benzoic acid of the formula III 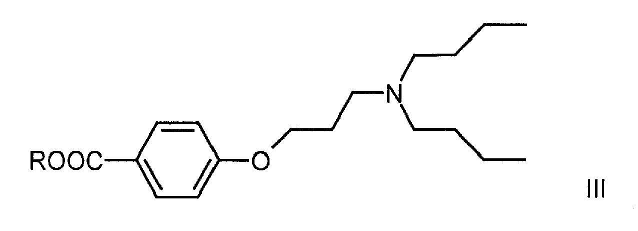 wherein R is defined as above;
b) deprotecting the compound of the formula III;
c) converting the deprotected compound obtain in step b) into 4-(3-dibutylaminopropoxy)benzoyl chloride of the formula IV 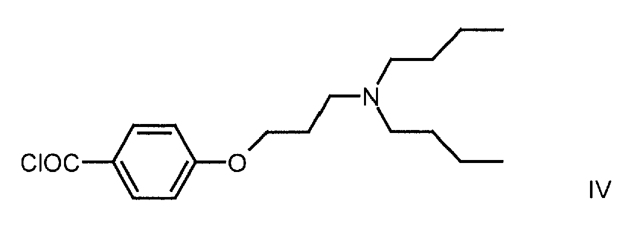 or a pharmaceutically acceptable salt thereof; and
d) reacting the compound of the formula IV or a pharmaceutically acceptable salt thereof with N-(2-butylbenzofuran-5-yl)methanesulfonamide of the formula V 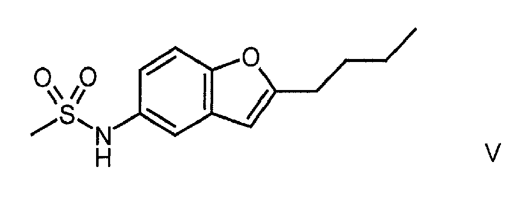 to obtain dronedarone or a pharmaceutically acceptable salt thereof.

5. Process according to any of claims 1-4, wherein the pharmaceutically acceptable salt is the hydrochloride salt.

6. Process according to any of claims 1-5, further comprising the step of essentially removing those impurities having a molecular weight being higher than the molecular weight of dronedarone free base.

7. Process according to claim 6, wherein the impurities having a molecular weight being higher than the molecular weight of dronedarone free base are removed by adding a solvent to the batch of dronedarone free base, dissolving the dronedarone free base in the solvent and removing the precipitated impurities.

8. Process for the preparation of crystalline dronedarone hydrochloride, which process comprises the purification process of claim 6 or 7 and the subsequent preparation of the crystalline dronedarone hydrochloride from the dronedarone free base obtained in the purification process.

9. Crystalline dronedarone hydrochloride.

10. Crystalline dronedarone hydrochloride according to claim 9, which is substantially free of impurities having a molecular weight being higher than the molecular weight of dronedarone free base.

11. Crystalline dronedarone hydrochloride according to claim 9 or 10, showing an XRPD pattern having characteristic peaks at 7.7 ± 0.2, 13.8 ± 0.2, 21.7 ± 0.2 and 26.2 ± 0.2 degree 2-Theta.

12. Crystalline dronedarone hydrochloride according to any of claims 9-11, showing an XRPD pattern essentially as in figure 1.

13. Pharmaceutical composition comprising a crystalline dronedarone hydrochloride according to any of claims 9-12.

14. Protected 4-(3-chloropropoxy)benzoic acid of the formula I 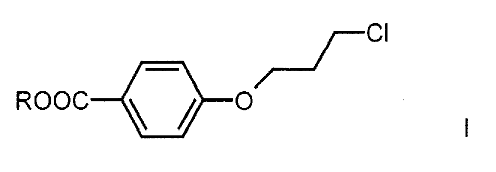 wherein R is a carboxyl protecting group.

15. Use of the protected 4-(3-chloropropoxy)benzoic acid of the formula I according to claim 14 in the preparation of dronedarone or a pharmaceutically acceptable salt thereof.
